# EUROPÄISCHE PATENTSCHRIFT

(11) **EP 2 298 519 B1**
(45) Veröffentlichungstag und Bekanntmachung des Hinweises auf die Patenterteilung: **15.05.2013**
(21) Anmeldenummer: 10197055.6
(22) Anmeldetag: 21.12.2005
(51) Int. Cl.: B28B 17/00, G01N 33/38, G01N 15/08

(54) **Vorrichtung zur Qualitätskontrolle eines Prozesses zur Herstellung von Betonwarenprodukten**
Device for controlling the quality of the production process of concrete products
Dispositif pour le contrôle de la qualité de procédé de production de produits en béton

(43) Veröffentlichungstag der Anmeldung: 23.03.2011
(62) Teilanmeldung aus: 05028086.6
(73) Patentinhaber: Qavertec GmbH, 24976 Flensburg-Handewitt (DE)
(72) Erfinder: Biallas, Thorsten, 24943 Flensburg (DE)
(74) Vertreter: UEXKÜLL & STOLBERG

(56) Entgegenhaltungen:
- DD-A1- 267 797
- DE-A1- 3 628 955
- DE-A1- 19 519 275
- GB-A- 2 179 588
- US-A- 5 766 538

## Beschreibung

Die vorliegende Erfindung betrifft eine Vorrichtung zur Qualitätskontrolle eines Prozesses zur Herstellung von Betonwarenprodukten.

Betonwarenprodukte werden als Fertigprodukte zu ihrem Einsatzort, wie etwa zu einer Baustelle, geliefert. Ein Beispiel für Betonwarenprodukte sind Pflastersteine. Zur Herstellung von Betonwarenprodukten wird zunächst Beton nach einer vorgegebenen Rezeptur gemischt. Anschließend wird der Beton in eine Maschine eingebracht, in der er in die Form der gewünschten Betonwarenprodukte gepresst und dabei verdichtet wird. Im Falle von Pflastersteinen wird eine sogenannte Steinformmaschine verwendet, um die Pflastersteine zu pressen. Die durch den Verdichtungsprozess entstehenden Rohlinge sind noch weich und verarbeitbar. Nach dem Verdichtungsprozess werden die Rohlinge für ungefähr ein bis zwei Tage in einer Trockenkammer angeordnet und anschließend für einen weiteren Zeitraum gelagert. In der Trockenkammer und während der anschließenden Lagerung erhärtet der Beton, und die Rohlinge werden auf diese Weise zu den fertigen Betonwarenprodukten.

Der Herstellungsprozess der Betonwarenprodukte kann grob in zwei Phasen eingeteilt werden. Die erste Phase bis zum Erhärten des Betons, d.h. die Phase, in der die Rohlinge noch weich und verarbeitbar sind, wird im allgemeinen als Frischbetonphase bezeichnet. Entsprechend werden die noch weichen und verarbeitbaren Rohlinge als Frischbetonprodukte bezeichnet. Die Frischbetonprodukte sind demnach die fertig gepressten und verdichteten Vorformen der fertigen Betonwarenprodukte. An die Frischbetonphase schließt sich die Festbetonphase an, in der der Beton erhärtet ist. Entsprechend werden die fertigen Betonwarenprodukte auch als Festbetonprodukte bezeichnet. Demnach wird zur Herstellung eines bestimmten Betonwarenproduktes zunächst aus dem in geeigneter Weise gemischten Beton ein entsprechendes Frischbetonprodukt hergestellt, das durch Aushärten zu einem Festbetonprodukt wird, das das gewünschte Betonwarenprodukt darstellt.

Betonwarenprodukte kommen regelmäßig in Bereichen zum Einsatz, in denen sie nicht nur starken mechanischen Belastungen, sondern auch weiteren äußeren Einflüssen, wie Wasser, Schnee, Licht, Hitze oder Frost, ausgesetzt sind. Als Baumaterialien müssen sie dennoch eine lange Lebensdauer aufweisen und ferner bestimmten Qualitätsnormen genügen. Daher ist es von großer Bedeutung, zuverlässig die Qualität der auf die obige Weise hergestellten Betonwarenprodukte bestimmen zu können. So wird häufig ein Festigkeitswert, die Rohdichte, die Frostbeständigkeit oder ein anderes Qualitätsmaß der fertigen Betonwarenprodukte gemessen, um die Qualität des Herstellungsprozesses und der durch diesen hergestellten Betonwarenprodukte zu bewerten und auf Basis dieser Bewertung gegebenenfalls Korrekturen an den Prozessparametern vorzunehmen.

Dabei tritt die Schwierigkeit auf, dass das Erhärten des Frischbetonproduktes zu dem Festbetonprodukt über einen relativ langen Zeitraum erfolgt. So erhält der Beton seine Festigkeit dadurch, dass die Klinkerbestandteile des einen Teil der Betonmischung bildenden Zements auskristallisieren. Durch das Auskristallisieren entstehen kleine Kristallnadeln, die sich fest ineinander verzahnen. Da sich das Kristallwachstum jedoch über einen Zeitraum von einigen Monaten fortsetzt, wird die endgültige Festigkeit erst lange nach dem Formen und Verdichten bzw. der Herstellung des Frischbetonproduktes erreicht. In der DIN 1164/EN 1338 ist die Normfestigkeit als genormtes Qualitätsmaß für die Festbetonprodukte als die Festigkeit definiert, die unter normalen Temperatur- und Feuchtigkeitsbedingungen nach 28 Tagen erreicht wird. In ähnlicher Weise werden auch aussagekräftige Werte weiterer Qualitätsmaße, wie zum Beispiel die Rohdichte oder die Frostbeständigkeit, erst nach diesem Zeitraum erreicht. Daher werden zur Qualitätskontrolle einzelne fertige Test-Betonwarenprodukte gewöhnlich erst nach 28 Tagen auf ihre Qualität überprüft. Erst dann wird festgestellt, ob die Betonwarenprodukte den gewünschten Anforderungen entsprechen oder nicht.

Die charakteristischen Eigenschaften der fertigen Betonwarenprodukte, wie zum Beispiel ihre Festigkeit, werden maßgeblich durch die Einsatzstoffe und ihre relativen Mengen in der anfänglichen Betonmischung sowie durch die Verdichtung bei der Herstellung des Rohlings bzw. Frischbetonproduktes bestimmt. So können bei der Verdichtung Verdichtungsunterschiede auftreten, die zu unterschiedlichen Güten führen. Dasselbe gilt für Variationen in den durch die gewählte Rezeptur vorgegebenen Rohstoffen. So kann insbesondere das auch als W/Z-Wert bezeichnete Wasser/Zement-Verhältnis, das durch direkt zugegebenes Wasser und in den übrigen Rohstoffen enthaltenes Wasser bestimmt wird, im Rahmen einer gegebenen Rezeptur schwanken. Daher ist die Frischbetonkonsistenz vor der Herstellung festzulegen und möglichst genau einzuhalten. Im Fall von Wasser werden regelmäßig Wasserdosiergeräte in den Mischern eingesetzt, um die Variation des Wassergehalts zu begrenzen.

Da Variationen in dem Herstellungsprozess aber nicht vollständig ausgeschlossen werden können, besteht aufgrund der oben angegebenen Umstände das Problem, dass die Qualität des Herstellungsprozesses erst einen Monat nach der Mischung und Verdichtung bewertet werden kann. Fehlproduktionen können daher erst lange nach dem Zeitpunkt festgestellt werden, zu dem noch steuernd in den Herstellungsprozess eingegriffen werden kann. Produktionsschwankungen werden teilweise zu spät bemerkt und es kommt zu Verlusten für das herstellende Unternehmen. Auch bei Versuchen oder Rezepturänderungen sind die Ergebnisse erst nach Tagen feststellbar.

Aus diesen Gründen sind im Stand der Technik vereinzelt Stichproben auf der Frischbetonseite durchgeführt worden, indem einzelne Frischbetonprodukte dem Herstellungsprozess entnommen und begutachtet wurden. So wurde vereinzelt manuell die Masse oder die Höhe von Frischbetonprodukten gemessen oder die Gefügestruktur untersucht und der jeweilige einzelne Messwert auf Grundlage von Erfahrungswerten beurteilt. In den meisten Fällen wurde für das Frischbetonprodukt subjektiv durch einen Maschinenführer entschieden, ob ein Produkt gut sein könnte oder nicht. Diese subjektive Beurteilung führt häufig zu erhöhten Materialkosten, da der Maschinenführer dazu neigt, die Mischung so zu korrigieren, dass kein Risikos einer Fehlproduktion eingegangen wird.

Aus US 5,766,538 ist eine Vorrichtung zur Qualitätskontrolle eines Prozesses zur Herstellung von Betonwarenprodukten bekannt, die angepasst ist, um Messungen an den Betonwarenprodukten in einem Zwischenschritt des Prozesses nach dem Formen und Verdichten in der Frischbetonphase durchzuführen, wobei die Vorrichtung eine Einrichtung zur Bestimmung eines Maßes für die Rohdichte eines Test-Frischbetonproduktes aus dem Zwischenschritt des Prozesses aufweist.

DD 267 797 A1 offenbart eine Vorrichtung zur Qualitätskontrolle eines Prozesses zur Herstellung von Betonwarenprodukten, die angepasst ist, um Messungen an den Betonwarenprodukten in einem Zwischenschritt des Prozesses nach dem Formen und Verdichten in der Frischbetonphase durchzuführen gemäß dem Oberbegriff des Anspruchs 1. Sie hat eine Einrichtung zur Bestimmung eines Maßes für die Rohdichte eines Test-Frischbetonproduktes aus dem Zwischenschritt des Prozesses und eine Einrichtung zur Bestimmung der Gaspermeabilität des Test-Frischbetonproduktes.

Die DE 36 28 955 A1 beschreibt eine Einrichtung mit einer Kammer, die eine Öffnung hat, deren Ränder dichtend in Anlage an einen Bereich der Außenfläche eines Test-Frischbetonproduktes gebracht werden kann, um die Öffnung zu schließen. Die Einrichtung ist zur Bestimmung der Gaspermeabilität eines Betonbauteils vorgesehen und weist eine Vakuumpumpe, mit der in der Kammer ein Unterdruck erzeugt werden kann, und ein Manometer zur Bestimmung eines Maßes für den durch die Druckdifferenz bewirkten Gasdurchfluss durch das Bauteil auf.

Es ist die Aufgabe der Erfindung, eine Vorrichtung zur Qualitätskontrolle eines Prozesses zur Herstellung von Betonwarenprodukten anzugeben, mit denen einfach, kostengünstig und schnell eine Bewertung der Qualität vorgenommen werden kann und die genannten Nachteile beseitigt werden. Ferner soll die Vorrichtung robust sein, um sie in einer rauen Produktionsumgebung betreiben zu können.

Zur Lösung dieser Aufgabe dienen die Merkmale des Patentanspruchs 1. Vorteilhafte Ausführungsformen der Vorrichtung sind Gegenstand der zugehörigen Unteransprüche.

Nach der vorliegenden Erfindung ist eine Vorrichtung vorgesehen, die angepasst ist, um Messungen an den Betonwarenprodukten in einem Zwischenschritt des Herstellungsprozesses nach dem Formen und Verdichten in der Frischbetonphase durchzuführen, und die in vorteilhafter Weise bei Durchführung eines Verfahrens zum Einsatz kommen kann, bei dem an einem Test-Frischbetonprodukt aus einem Zwischenschritt des Herstellungsprozesses nach dem Formen und Verdichten Messungen vorgenommen werden, um Messwerte zu ermitteln, auf deren Grundlage durch ein geeignetes Vorhersagemodell ein Wert für ein Qualitätsmaß des Festbetonproduktes abgeschätzt werden kann, das sich im Verlaufe des weiteren Herstellungsprozesses aus dem vermessenen Test-Frischbetonprodukt ergeben würde. Mit anderen Worten wird bei diesem Verfahren auf Grundlage von Messungen, die in der Anfangsphase des Herstellungsprozesses durchgeführt werden, ein Wert bestimmt, der unmittelbar erst nach Abschluss des Herstellungsprozesses und damit erst sehr viel später gemessen werden kann. Das Test-Frischbetonprodukt wird einfach an geeigneter Stelle dem Herstellungsprozess entnommen. Es kann zum Beispiel zufällig als Stichprobe ausgewählt werden.

Die für das Test-Frischbetonprodukt bestimmten Messwerte umfassen dabei zumindest ein Maß für die Rohdichte und die Gaspermeabilität des Test-Frischbetonproduktes. Als Maß für die Rohdichte des Test-Frischbetonproduktes wird bevorzugt eine geometrische Abmessung und die Masse des Test-Frischbetonproduktes gemessen. Werden eine geometrische Abmessung und die Masse gemessen, so stellt das Wertepaar aus geometrischer Abmessung und Masse das Maß für die Rohdichte dar. Eine geometrische Abmessung bzw. die Ausdehnung des Test-Frischbetonproduktes in einer bestimmten Erstreckungsrichtung, wie zum Beispiel die Höhe, ist insbesondere dann zusammen mit der Masse zur Bestimmung der Rohdichte des Frischbetonproduktes besonders genau, wenn die weiteren geometrischen Abmessungen des Frischbetonproduktes, wie zum Beispiel die Breite und die Tiefe, als Sollwerte vorgegeben werden. Dies ist häufig möglich, da die Verdichtung regelmäßig nur aus einer Richtung erfolgt, während die weiteren Seiten durch eine geeignete Form vorgegeben sind. So wird regelmäßig von oben verdichtet, während von unten eine Vibration eingeführt wird und die Seiten starr geschlossen sind. Die gemessene geometrische Abmessung sollte bevorzugt in der Richtung liegen, in der die Verdichtung erfolgt ist. Es ist aber natürlich auch möglich, die geometrischen Abmessungen des Test-Frischbetonproduktes vollständig zu bestimmen, d.h. im Falle von quaderförmigen Produkten die Höhe, Breite und Tiefe. Dann bilden die Masse und alle geometrischen Abmessungen zusammen das Maß für die Rohdichte.

Mit Hilfe dieser Messwerte kann anhand eines vorbestimmten Vorhersagemodells, das die Messwerte von Frischbetonprodukten aus dem Zwischenschritt des Prozesses mit dem Qualitätsmaß der entsprechenden Festbetonprodukte in Beziehung setzt, ein Schätzwert für ein Qualitätsmaß des Festbetonproduktes bestimmt werden, das sich im weiteren Prozessverlauf aus dem Test-Frischbetonprodukt ergibt bzw. ergeben würde. Das Vorhersagemodell muss prinzipiell für jede Rezeptur und für gegebene Prozessparameter angepasst werden und bezieht sich stets auf einen bestimmten Zwischenschritt des Prozesses, so dass das Test-Frischbetonprodukt mit ausreichender Genauigkeit derselben Stelle des Prozesses entnommen werden sollte. Abweichungen führen zu einer verringerten Genauigkeit. Daher sind Abweichungen in dem Maße zulässig, in dem die gewünschte Genauigkeit nicht unterschritten wird. Im Extremfall ist es auch möglich ein einziges Vorhersagemodell für alle Rezepturen bereitzustellen. Ein solches Verfahren ist besonders einfach durchzuführen, hat aber den Nachteil, dass die Genauigkeit der Schätzwerte unter Umständen erheblich verringert ist. Dennoch kann ein solches allgemeines Vorhersagemodell in bestimmten Anwendungen von Vorteil sein.

Die Gaspermeabilität ist ein Maß für die Gefügewerte oder auch die Nutzporosität des Test-Frischbetonproduktes. Die Gaspermeabilität ist als Permeabilität für ein bestimmtes Gas oder Gasgemisch zu verstehen, da sich die Permeabilität im allgemeinen für verschiedene Gase bzw. Gasgemische unterscheidet. Für die Bestimmung des Vorhersagemodells und die späteren Messungen muss daher im allgemeinen stets dasselbe Gas bzw. Gasgemisch verwendet werden. Das genaue Messverfahren ist nicht von Bedeutung. Es kommt nur darauf an, dass stets dasselbe Messverfahren in demselben Verfahrensschritt, d.h. an vergleichbaren Proben, durchgeführt wird. So muss auch stets dieselbe geometrische Abmessung bestimmt werden.

Im Rahmen der vorliegenden Erfindung wurde herausgefunden, dass die obigen zwei (Rohdichte und Gaspermeabilität) bzw. drei (geometrische Abmessung, Masse und Gaspermeabilität) Messwerte ausreichen, um ein derartiges Vorhersagemodell zu erstellen. Es ist daher möglich, aus mehreren kombinierten Messverfahren bereits in der Frischbetonphase eine Aussage über die spätere Güte des Endproduktes zu treffen. Dadurch wird die vorteilhafte Möglichkeit geschaffen, frühzeitig drohende Fehlproduktionen zu erkennen und gegenzusteuern. Nach Vermessung eines in definierter Weise hergestellten Frischbetonproduktes mit bekannten und definierten Sollmaßen kann sofort entschieden werden, ob der Rohling später beispielsweise die nötige Festigkeit erreicht oder nicht. Die Qualität von Betonwarenprodukten, wie z.B. Pflastersteinen, kann in vorteilhafter Weise direkt nach den die Endqualität bestimmenden Produktionsschritten bestimmt werden. Auf diese Weise kann der Hersteller seine Produktion effektiv und normgerecht steuern. Eine mögliche Fehlproduktion kann sofort ausgeschlossen werden. Die bisherige Überwachungslücke auf der Frischbetonseite wird geschlossen, und spätere Prüfergebnisse des Festbetons können nicht mehr zu unangenehmen Überraschungen führen. Es wird Sicherheit bei der Produktion und Entwicklung veränderter Rezepturen geschaffen, vor möglichen Fehlern gewarnt und Zeit und Geld gespart.

Die erfindungsgemäße Vorrichtung weist eine Einrichtung zur Bestimmung eines Maßes für die Rohdichte des Test-Frischbetonproduktes aus dem Zwischenschritt des Prozesses und eine Einrichtung zur Bestimmung der Gaspermeabilität des Test-Frischbetonproduktes auf. Die Einrichtung zur Bestimmung eines Maßes für die Rohdichte des Test-Frischbetonproduktes weist dabei bevorzugt eine Einrichtung zur Bestimmung einer geometrischen Abmessung des Test-Frischbetonproduktes und eine Einrichtung zur Bestimmung der Masse des Test-Frischbetonproduktes auf. Das von diesen letzteren Einrichtungen jeweils gelieferte Wertepaar stellt dann das Maß für die Rohdichte dar. Es ist auch möglich, dass die Einrichtung zur Bestimmung eines Maßes für die Rohdichte weitere Messeinrichtungen enthält, die weitere oder alle geometrischen Abmessungen bestimmt, wobei die weiteren Abmessungen dann in das Maß für die Rohdichte eingezogen werden.

In der Vorrichtung werden somit mehrere Messeinrichtungen kombiniert, mit deren Hilfe eine Abschätzung der Qualität des Endproduktes möglich ist.

Diese Abschätzung kann manuell oder in einer gesonderten Recheneinrichtung erfolgen, in die die von der Vorrichtung gelieferten oder angezeigten Messwerte eingegeben werden. Alternativ kann die Vorrichtung eine Auswerteeinrichtung aufweisen, die mit der Einrichtung zur Bestimmung eines Maßes für die Rohdichte und der Einrichtung zur Bestimmung der Gaspermeabilität bzw. mit der Einrichtung zur Bestimmung einer geometrischen Abmessung, der Einrichtung zur Bestimmung der Masse und der Einrichtung zur Bestimmung der Gaspermeabilität gekoppelt und angepasst ist, um die von diesen gelieferten Messwerte für ein Test-Frischbetonprodukt aus dem Zwischenschritt des Prozesses zu empfangen und anhand eines vorbestimmten Vorhersagemodells, das die Messwerte von Frischbetonprodukten aus dem Zwischenschritt des Prozesses mit einem Qualitätsmaß der entsprechenden Festbetonprodukte in Beziehung setzt, einen geschätzten Wert für das Qualitätsmaß des Festbetonproduktes zu ermitteln, das sich im weiteren Prozessverlauf aus dem vermessenen Test-Frischbetonprodukt ergibt bzw. ergeben würde. In diesem Fall kann die Vorrichtung selbst ein Signal liefern, das angibt, ob die angestrebte Qualität mit den gegenwärtigen Prozessparametern erreicht wird. Das Signal kann auf einer geeigneten Anzeigeeinrichtung der Vorrichtung angezeigt werden. Eine besonders einfache Anzeigeeinrichtung besteht aus Lampen unterschiedlicher Farben, wobei beispielsweise eine grüne Lampe eine ausreichende Qualität und eine rote Lampe eine ungenügende Qualität anzeigt.

Ferner weist die Einrichtung zur Bestimmung der Gaspermeabilität eine Kammer, die eine Öffnung hat, deren Ränder dichtend in Anlage an einen Bereich der Außenfläche des Test-Frischbetonproduktes gebracht werden kann, um die Öffnung zu schließen, eine Druckerzeugungseinrichtung, mit der in der Kammer ein Überdruck oder ein Unterdruck erzeugt werden kann, und eine Einrichtung zur Bestimmung eines Maßes für den durch die Druckdifferenz bewirkten Gasdurchfluss durch das Test-Frischbetonprodukt auf. Mit anderen Worten kann mit der Druckerzeugungseinrichtung ein Überdruck oder ein Unterdruck in einem Raum hergestellt werden, der in der Messposition einseitig durch das Test-Frischbetonprodukt geschlossen wird. Die Einrichtung zur Bestimmung der Gaspermeabilität kann auch in vorteilhafter Weise mehrere separate dieser Kammern aufweisen, so dass es möglich ist, ein inhomogenes Betonwarenprodukt zu vermessen. So weisen beispielsweise Pflastersteine im Allgemeinen eine Kernkomponente und eine Vorsatzkomponente auf, die aus unterschiedlichem Beton bestehen. Mit zwei Messkammern kann dann bei geeigneter Anordnung die Gaspermeabilität separat für die beiden Komponenten bestimmt werden.

Auf diese Weise kann die Gaspermeabilität gemessen werden, indem entlang der Außenfläche des Test-Frischbetonproduktes eine Druckdifferenz hergestellt und ein Maß für den durch die Druckdifferenz bewirkten Gasdurchfluss durch das Test-Frischbetonprodukt bestimmt wird. Mit anderen Worten wird ein Bereich der Oberfläche des Test-Frischbetonproduktes mit einem Überdruck oder einem Unterdruck beaufschlagt und eine Größe gemessen, die für das durch das Test-Frischbetonprodukt gepresste oder gesogene Gas charakteristisch ist. Die Gaspermeabilität ist nur von dem verwendeten Gas und den Eigenschaften des Frischbetonproduktes abhängig, wenn bei der Messung laminare Fließbedingungen eingehalten werden und keine oder nur eine geringe Wechselwirkung zwischen dem Frischbetonprodukt und dem Gas besteht.

Schließlich ist die Vorrichtung so aufgebaut, dass das Test-Frischbetonprodukt in einer einzigen Messposition angeordnet werden kann, in der gleichzeitig alle erforderlichen Messwerte ermittelt werden können. Dabei wird das Test-Frischbetonprodukt durch Unterdruck in der Messposition fixiert, um eine schnelle und robuste Messung durchführen zu können.

Das Verfahren, bei dessen Durchführung die Vorrichtung vorteilhaft zum Einsatz kommen kann, kann beispielsweise die folgenden weiteren Merkmale aufweisen.

Es ist besonders vorteilhaft, wenn die geometrische Abmessung durch ein optisches Messverfahren gemessen wird. Dem Fachmann sind eine Vielzahl geeigneter Messverfahren bekannt. Mit optischen Messverfahren kann der entsprechende Messwert sehr schnell und einfach erhalten werden. Eine besonders hohe Genauigkeit lässt sich erreichen, wenn die geometrische Abmessung mit Hilfe eines Lasers gemessen wird.

Im Falle eines quaderförmigen Betonwarenproduktes, wie zum Beispiel eines Pflastersteins, ist es bevorzugt, wenn die geometrische Abmessung die Höhe des Test-Frischbetonproduktes ist. Dabei bezieht sich der Begriff Höhe auf die später vorgesehene Einbaulage des Betonwarenproduktes.

Weiterhin ist es bevorzugt, wenn die Gaspermeabilität die Permeabilität gegenüber Luft ist. Diese Messung lässt sich auf besonders einfache, schnelle und kostengünstige Weise realisieren, da mit der normalen Atmosphäre gearbeitet werden kann und der Einsatz besonderer Gase nicht erforderlich ist. Es ist aber zu bedenken, dass in diesem Fall für die Genauigkeit der Messung eine obere Grenze existiert, da Beton gegenüber dem in der Luft enthaltenen Kohlendioxid nicht inert ist. Reicht die Genauigkeit der Messung mit Luft nicht aus, so können Gase wie Sauerstoff oder Stickstoff verwendet werden.

Es ist insbesondere von Vorteil, wenn die Druckdifferenz erzeugt wird, indem ein Bereich (oder mehrere separate Bereiche) der Oberfläche des Test-Frischbetonproduktes mit einem Unterdruck beaufschlagt wird (oder werden). Wird zur Erzeugung und Aufrechterhaltung des Unterdrucks eine Vakuumpumpe verwendet, die mit einer definierten Pumpleistung betrieben wird, kann die für den Gasdurchfluss charakteristische Größe in vorteilhafter Weise durch den durch die Vakuumpumpe erreichten Unterdruck gebildet werden. Je höher bei definierter Pumpleistung der Gasdurchfluss ist, desto schlechter ist das durch die Vakuumpumpe erreichte Vakuum. Alternativ kann die für den Gasdurchfluss charakteristische Größe in vorteilhafter Weise auch durch die Zeitkonstante gebildet werden, mit der der Druck nach Erzeugung eines definierten Unterdrucks und Deaktivierung der Vakuumpumpe ansteigt. Dabei erfolgt der Druckanstieg umso schneller, je höher der Gasdurchfluss ist. Wenn die Druckdifferenz erzeugt wird, indem ein Bereich der Oberfläche des Test-Frischbetonproduktes mit einem Unterdruck beaufschlagt wird, ist es ferner in vorteilhafter Weise möglich, den Unterdruck dazu zu verwenden, um das Test-Frischbetonprodukt anzuheben und zu transportieren.

Das Vorhersagemodell wird bevorzugt empirisch anhand eines Satzes von Kalibrierungs-Frischbetonprodukten ermittelt, die dem Herstellungsprozess in dem Zwischenschritt vorübergehend entnommen und anschließend wieder in den Herstellungsprozess eingeführt werden, um aus ihnen Festbetonprodukte zu bilden. Dabei werden für die Kalibrierungs-Frischbetonprodukte zunächst die obigen Messwerte, d.h. die Rohdichte und die Gaspermeabilität bzw. die geometrische Abmessung, die Masse und die Gaspermeabilität, bestimmt. Nach der Herstellung der Festbetonprodukte aus den einzelnen Kalibrierungs-Frischbetonprodukten, d.h. nach 28 Tagen, wird für jedes dieser Festbetonprodukte der Wert für das Qualitätsmaß bestimmt. Anschließend kann durch geeignete Verfahren, wie zum Beispiel bekannte Regressionsverfahren, ein Zusammenhang zwischen den Werten für das Qualitätsmaß und den Messwerten ermittelt werden, der das Vorhersagemodell darstellt.

Das Vorhersagemodell kann auch in vorteilhafter Weise empirisch anhand von zwei Sätzen von Kalibrierungs-Frischbetonproben ermittelt werden, die repräsentativ für den Zwischenschritt des Herstellungsprozesses sind. Die Sätze sind so gewählt, dass für jedes Kalibrierungs-Frischbetonprodukt des einen Satzes ein Kalibrierungs-Frischbetonprodukt in dem anderen Satz vorhanden ist, das in genau derselben Weise hergestellt ist. Die einzelnen Sätze entstammen derselben Charge und werden mit derselben Mischung hergestellt. Die einander paarweise zugeordneten Frischbetonprodukte entstammen ferner bevorzugt derselben Position in der Formmaschine, um Verdichtungsunterschiede auszuschließen. Der eine Satz von Kalibrierungs-Frischbetonprodukten wird dem Herstellungsprozess in dem Zwischenschritt entnommen und nach der Bestimmung der obigen Messwerte entsorgt. Ferner wird für jedes der Festbetonprodukte, die sich aus den Kalibrierungs-Frischbetonprodukten des anderen Satzes ergeben, der Wert für das Qualitätsmaß bestimmt. Anschließend kann durch geeignete Verfahren, wie zum Beispiel bekannte Regressionsverfahren, ein Zusammenhang zwischen den Werten für das Qualitätsmaß und den Messwerten ermittelt werden, der das Vorhersagemodell darstellt.

In einer bevorzugten Ausführungsform ist das Qualitätsmaß die Rohdichte, die Frostbeständigkeit oder ein Festigkeitswert des Festbetonproduktes. Der Festigkeitswert kann beispielsweise eine Biegezugfestigkeit oder eine Druckfestigkeit sein.

Durch die Möglichkeit, die Qualität der Endprodukte bereits in einer Frühphase des Herstellungsprozesses zu bestimmen, ist es in vorteilhafter Weise möglich, den Prozess zur Herstellung von Betonwarenprodukten auf Basis der Differenz des anhand des Vorhersagemodells für das Test-Frischbetonprodukt bestimmten Wertes für das Qualitätsmaß des entsprechenden Festbetonproduktes und einem Sollwert für das Qualitätsmaß zu steuern. Es kann ein geschlossener Regelkreis gebildet werden, in dem die Prozessparameter automatisch so geändert werden, dass die Differenz zwischen Schätzwert und Sollwert minimiert wird.

Einige vorteilhafte Ausführungsbeispiele des Verfahrens sind im Folgenden zusammengefasst:

Beispiel 1: Verfahren zur Qualitätskontrolle eines Prozesses zur Herstellung von Betonwarenprodukten, wobei die Qualitätskontrolle anhand von Messungen erfolgt, die an den Betonwarenprodukten in einem Zwischenschritt des Prozesses nach dem Formen und Verdichten in der Frischbetonphase vorgenommen werden, dadurch gekennzeichnet, dass das Verfahren die folgenden Schritte aufweist: Bestimmen von Messwerten an einem Test-Frischbetonprodukt aus dem Zwischenschritt des Prozesses, wobei die Messwerte ein Maß für die Rohdichte und die Gaspermeabilität des Test-Frischbetonproduktes umfassen, und Bestimmen eines geschätzten Wertes für ein Qualitätsmaß des Festbetonproduktes, das sich im weiteren Prozessverlauf aus dem Test-Frischbetonprodukt ergibt, anhand eines vorbestimmten Vorhersagemodells, das die Messwerte von Frischbetonprodukten aus dem Zwischenschritt des Prozesses mit dem Qualitätsmaß der entsprechenden Festbetonprodukte in Beziehung setzt.

Beispiel 2: Bei einem Verfahren gemäß Beispiel 1 wird als Maß für die Rohdichte des Test-Frischbetonproduktes eine geometrische Abmessung und die Masse des Test-Frischbetonproduktes bestimmt.

Beispiel 3: Bei einem Verfahren gemäß Beispiel 2 wird die geometrische Abmessung mit Hilfe eines optischen Messverfahrens bestimmt.

Beispiel 4: Bei einem Verfahren gemäß Beispiel 3 wird die geometrische Abmessung mit Hilfe eines Lasers gemessen.

Beispiel 5: Bei einem Verfahren gemäß einem der Beispiel 2 bis 4 ist das Betonwarenprodukt quaderförmig und die geometrische Abmessung die Höhe des Test-Frischbetonproduktes.

Beispiel 6: Bei einem Verfahren gemäß einem der Beispiele 1 bis 5 ist die Gaspermeabilität die Permeabilität gegenüber Luft.

Beispiel 7: Bei einem Verfahren gemäß einem der Beispiele 1 bis 6 wird die Gaspermeabilität gemessen, indem entlang der Außenfläche des Test-Frischbetonproduktes eine Druckdifferenz hergestellt und ein Maß für den durch die Druckdifferenz bewirkten Gasdurchfluss durch das Test-Frischbetonprodukt bestimmt wird.

Beispiel 8: Bei einem Verfahren gemäß Beispiel 7 wird die Druckdifferenz erzeugt, indem ein Bereich der Oberfläche des Test-Frischbetonproduktes mit einem Unterdruck beaufschlagt wird.

Beispiel 9: Bei einem Verfahren gemäß Beispiel 8 wird der Unterdruck mit Hilfe einer Vakuumpumpe mit vorbestimmter Pumpleistung erzeugt und aufrecht erhalten, und als Maß für den Gasdurchfluss wird der durch die Vakuumpumpe erreichte Unterdruck gemessen.

Beispiel 10: Bei einem Verfahren gemäß Beispiel 8 wird der Unterdruck mit Hilfe einer Vakuumpumpe erzeugt, und als Maß für den Gasdurchfluss wird die Geschwindigkeit des Druckanstiegs nach Deaktivierung der Vakuumpumpe gemessen.

Beispiel 11: Bei einem Verfahren gemäß einem der Beispiele 8 bis 10 wird der Unterdruck verwendet, um das Test-Frischbetonprodukt anzuheben und zu transportieren.

Beispiel 12: Bei einem Verfahren gemäß einem der Beispiele 1 bis 11 wird das Vorhersagemodell empirisch ermittelt, indem die Messwerte für einen Satz von Kalibrierungs-Frischbetonprodukten aus dem Zwischenschritt des Prozesses bestimmt werden, für jedes der Festbetonprodukte, die sich aus den Kalibrierungs-Frischbetonprodukten ergeben, der Wert für das Qualitätsmaß bestimmt wird und ein Zusammenhang zwischen den Werten für das Qualitätsmaß und den Messwerten ermittelt wird.

Beispiel 13: Bei einem Verfahren gemäß einem der Beispiele 1 bis 11 wird das Vorhersagemodell empirisch ermittelt, indem zwei Sätze von Kalibrierungs-Frischbetonprodukten aus dem Zwischenschritt des Prozesses bereitgestellt werden, wobei für jedes Kalibrierungs-Frischbetonprodukt des einen Satzes ein Kalibrierungs-Frischbetonprodukt in dem anderen Satz vorhanden ist, das in genau derselben Weise hergestellt ist, und indem die Messwerte für einen Satz von Kalibrierungs-Frischbetonprodukten aus dem Zwischenschritt des Prozesses bestimmt werden und für jedes der Festbetonprodukte, die sich aus den Kalibrierungs-Frischbetonprodukten des anderen Satzes ergeben, der Wert für das Qualitätsmaß bestimmt wird und ein Zusammenhang zwischen den Werten für das Qualitätsmaß und den Messwerten ermittelt wird.

Beispiel 14: Bei einem Verfahren gemäß einem der Beispiele 1 bis 13 ist das Qualitätsmaß die Rohdichte, die Frostbeständigkeit oder ein Festigkeitswert des Festbetonproduktes.

Beispiel 15: Bei einem Verfahren gemäß einem der Beispiele 1 bis 14 wird der Prozess zur Herstellung von Betonwarenprodukten auf Basis der Differenz des anhand des Vorhersagemodells für das Test-Frischbetonprodukt bestimmten Wertes für das Qualitätsmaß des entsprechenden Festbetonproduktes und einem Sollwert für das Qualitätsmaß gesteuert.

Dementsprechend ist die erfindungsgemäße Vorrichtung in vorteilhaften Ausführungsformen wie folgt weiter ausgestaltet.

In einer bevorzugten Ausführungsform ist die Einrichtung zur Bestimmung einer geometrischen Abmessung eine optische Messeinrichtung, mit der die geometrische Abmessung mit einem geeigneten optischen Messverfahren gemessen werden kann. Dem Fachmann sind eine Vielzahl geeigneter Messeinrichtungen und Messverfahren bekannt. Mit optischen Messverfahren kann der entsprechende Messwert sehr schnell und einfach erhalten werden. Eine besonders hohe Genauigkeit lässt sich erreichen, wenn die Einrichtung zur Bestimmung einer geometrischen Abmessung einen Laser aufweist, mit dessen Hilfe die geometrische Abmessung bestimmt werden kann.

In einer weiteren bevorzugten Ausführungsform ist die Einrichtung zur Bestimmung einer geometrischen Abmessung angepasst, um die Höhe eines quaderförmigen Betonwarenproduktes zu bestimmen. Dabei bezieht sich der Begriff Höhe auf die später vorgesehene Einbaulage des Betonwarenproduktes.

Weiterhin ist es bevorzugt, wenn die Einrichtung zur Bestimmung der Gaspermeabilität angepasst ist, um die Permeabilität gegenüber Luft zu bestimmen. Eine solche Messeinrichtung lässt sich auf besonders einfache und kostengünstige Weise realisieren, da mit der normalen Atmosphäre gearbeitet werden kann und der Einsatz besonderer Gase nicht erforderlich ist. Die Messung kann sehr schnell mit hoher Genauigkeit durchgeführt werden.

Es ist insbesondere bevorzugt, wenn die Druckerzeugungseinrichtung angepasst ist, um in der durch das Test-Frischbetonprodukt abgeschlossenen Kammer einen Unterdruck zu erzeugen. In diesem Fall umfasst die Druckerzeugungseinrichtung bevorzugt eine Vakuumpumpe, und die Einrichtung zur Bestimmung der Gaspermeabilität weist eine Druckmesseinrichtung zur Bestimmung des Druckes in der Kammer auf. Wird die Vakuumpumpe mit einer definierten Pumpleistung betrieben, so hängt der von der Druckmesseinrichtung gemessene Druck von dem durch den Unterdruck bewirkten Gasdurchfluss durch das Test-Frischbetonprodukt ab. In einer weiteren bevorzugten Ausgestaltung ist ferner eine Zeitmesseinrichtung vorgesehen, mit der die Geschwindigkeit des Druckanstiegs nach Deaktivierung der Vakuumpumpe gemessen werden kann. Diese Zeitkonstante von einem definierten Druckniveau aus ist ein einfach und genau zu bestimmendes Maß für den Gasdurchfluss, da es nicht auf die genaue absolute Messung eines Drucks ankommt und daher geringere Anforderungen an die Druckmesseinrichtung gestellt werden.

Es ist von Vorteil, wenn eine Transporteinrichtung zum Anheben und Transportieren des Test-Frischbetonproduktes vorgesehen ist, wobei die Kammer einen Teil der Transporteinrichtung bildet und das Test-Frischbetonprodukt angehoben und transportiert werden kann, wenn in der Kammer ein Unterdruck erzeugt wird. Eine solche Transporteinrichtung lässt sich besonders einfach und kostengünstig realisieren. Mit der Transporteinrichtung kann das Test-Frischbetonprodukt aus dem Herstellungsprozess entnommen, in eine Messposition gebracht und entsorgt oder wieder in den Herstellungsprozess eingefügt werden.

Es ist weiterhin bevorzugt, wenn die Auswerteeinrichtung einen Speicher aufweist, in dem die Messwerte für einen Satz von Kalibrierungs-Frischbetonprodukten aus dem Zwischenschritt des Prozesses gespeichert werden können, und wenn die Auswerteeinrichtung ferner eine Eingabeeinrichtung zur Eingabe von Werten für das Qualitätsmaß für jedes der Festbetonprodukte aufweist, die sich aus den Kalibrierungs-Frischbetonprodukten ergeben, oder für Festbetonprodukte, die sich aus einem zweiten Satz von Kalibrierungs-Frischbetonprodukten ergeben, der in derselben Weise hergestellt wurde wie der erste Satz. Dabei ist die Auswerteeinrichtung angepasst, um die eingegebenen Werte für das Qualitätsmaß in dem Speicher zu speichern und einen Zusammenhang zwischen den Werten für das Qualitätsmaß und den Messwerten zu ermitteln.

In dem Speicher können auch die verschiedenen Vorhersagemodelle sowie weitere für ein bestimmtes Betonwarenprodukt spezifische Parameter gespeichert werden. Diese Parameter können beispielsweise Sollwerte für die Breite und die Tiefe eines quaderförmigen Betonwarenproduktes sein. Es ist dann später möglich, an der Auswerteeinrichtung das für ein bestimmtes Betonwarenprodukt passende Vorhersagemodell und die passenden weiteren Parameter auszuwählen.

Die Erfindung wird im Folgenden anhand eines Ausführungsbeispiels, das in der Zeichnung dargestellt ist, näher erläutert.

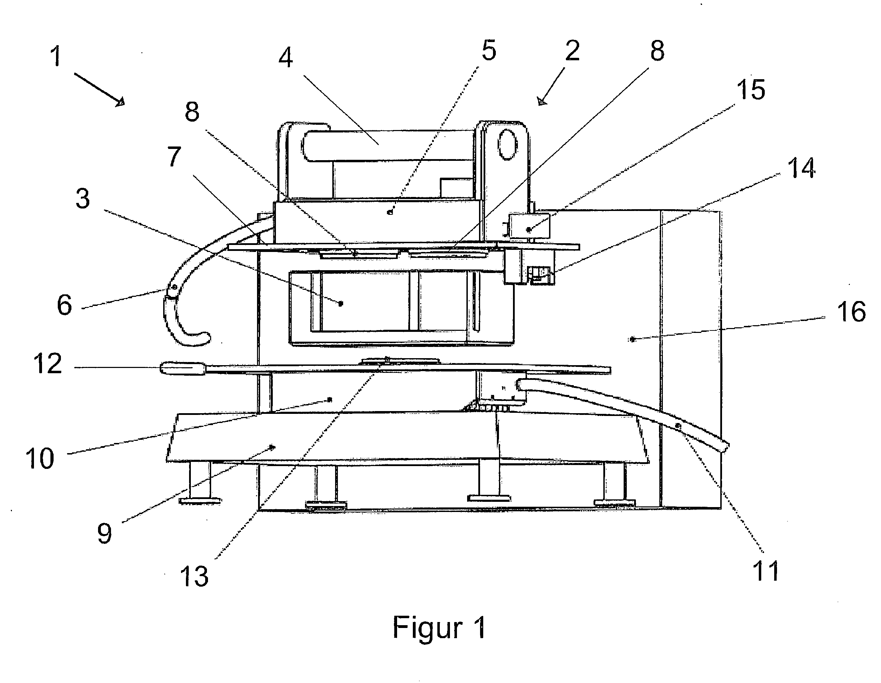

- Figur 1: zeigt eine perspektivische schematische Ansicht einer erfindungsgemäßen Vorrichtung zur Qualitätskontrolle eines Prozesses zur Herstellung von Betonwarenprodukten.

Die in Figur 1 gezeigte Vorrichtung 1 zur Qualitätskontrolle eines Prozesses zur Herstellung von Betonwarenprodukten weist eine Transporteinrichtung 2 zum Anheben und Transportieren eines Frischbeton-Pflastersteins 3 auf. Die Transporteinrichtung 2 kann von einer Bedienungsperson mit der Hand an einem Handgriff 4 ergriffen werden, so dass der Pflasterstein 3 von der Bedienungsperson per Hand angehoben und in die Messposition in der Vorrichtung 1 transportiert werden. Bei geeigneter Anordnung der Vorrichtung 1 kann ein Pflasterstein 3 mit Hilfe der Transporteinrichtung 2 zur Messung unmittelbar aus dem Herstellungsprozess herausgenommen werden.

Zum Festhalten des Pflastersteins 3 weist die Transporteinrichtung 2 eine Basis 5 auf, deren Innenraum (nicht gezeigt) über eine Leitung 6 zur Erzeugung eines Unterdrucks (teilweise) evakuiert werden kann. Zu diesem Zweck ist die Leitung 6 mit einer Vakuumpumpe (nicht gezeigt) verbunden. In einer unteren Abschlussplatte 7 der Basis 5 der Transporteinrichtung 2 sind zwei von einer geeigneten Abdichtung umgebene Öffnungen 8 vorgesehen, die mit ihren Abdichtungen auf die Oberseite des Pflastersteins 3 aufgelegt werden können, um den Innenraum der Basis 5 durch den Pflasterstein 3 zu verschließen. Auf diese Weise wird der Pflasterstein 3 nach Evakuierung des Innenraums der Basis 5 durch den Unterdruck an der Transporteinrichtung 2 festgehalten. Das Vorsehen von zwei Öffnungen 8 hat gegenüber dem ebenfalls möglichen Vorsehen nur einer Öffnung den Vorteil, dass die Messung aufgrund der Verteilung der Öffnungen repräsentativer ist und dass durch die Verkleinerung der Einzelöffnungen die Gefahr einer Verformung des vermessenen Frischbetonprodutes minimiert wird.

Die Vorrichtung 1 weist ferner eine Waage 9 auf, auf der sich eine Messeinheit 10 befindet. Der Innenraum der Messeinheit 10 kann zur Erzeugung eines Unterdrucks über eine Leitung 11 (teilweise) evakuiert werden. Zu diesem Zweck ist die Leitung 11 genau wie die Leitung 6 mit einer Vakuumpumpe (nicht gezeigt) verbunden. Dabei können die Leitungen 6 und 11 mit derselben oder mit verschiedenen Vakuumpumpen verbunden sein. In einer oberen Abschlussplatte 12 der Messeinheit 10 ist eine von einer geeigneten Abdichtung umgebene Öffnung 13 vorgesehen, auf deren Abdichtung der von der Transporteinrichtung 2 festgehaltene Pflasterstein 3 aufgelegt werden kann, um den Innenraum der Messeinheit 10 durch den Pflasterstein 3 zu verschließen. Auf diese Weise wird der Pflasterstein 3 nach Evakuierung des Innenraums der Messeinheit 10 durch den Unterdruck an der Messeinheit 10 festgehalten.

Dieser Zustand, in dem der Pflasterstein 3 mittels Unterdruck sowohl von der Transporteinrichtung 2 als auch von der Messeinheit 10 festgehalten wird, ist die Messposition des Pflastersteins 3. In dieser ist der Pflasterstein 3 während der Messung sicher fixiert. Dadurch können die Messungen auch in einer rauen Produktionsumgebung zuverlässig und in einfacher Weise durchgeführt werden. Dabei kann nicht nur die Masse des Pflastersteins 3 mit Hilfe der Waage 9 bestimmt werden, sondern es können parallel mehrere unterschiedliche weitere Messungen durchgeführt und die entsprechenden Messwerte ermittelt werden.

Zur Bestimmung der Höhe des Pflastersteins 3 ist an der Transporteinrichtung 2 ein optischer Entfernungssensor 14 angebracht, der im Falle des Wunsches einer hohen Genauigkeit zum Beispiel ein Laser-Entfernungssensor sein kann und der als Maß für die Höhe die Entfernung zu der Abschlussplatte 12 der Messeinheit 10 misst. Ferner ist an der Transporteinrichtung 2 eine Druckmesseinrichtung 15 angeordnet, mit der der Druck in dem Innenraum der Basis 5 der Transporteinrichtung 2 gemessen werden kann. In ähnlicher Weise ist in der Messeinheit 10 eine Druckmesseinrichtung (nicht gezeigt) vorgesehen, mit der der Druck in dem Innenraum der Messeinheit 10 gemessen werden kann. Wird die Vakuumpumpe bzw. werden die Vakuumpumpen mit einer definierten Pumpleistung betrieben, so sind die von den Druckmesseinrichtungen der Transporteinrichtung 2 und der Messeinheit 10 gemessenen Drücke charakteristisch für den durch die Unterdrücke bewirkten Luftdurchflüsse durch den Pflasterstein 3.

Die von der Druckmesseinrichtung 15 und von dem Laser-Entfernungssensor 14 gelieferten Signale werden über elektrische Leitungen, die in die Leitung 6 integriert sein können, oder drahtlos an eine Auswerte- und Steuereinheit 16 geliefert. In ähnlicher Weise werden die durch die Waage 9 und die Druckmesseinrichtung der Messeinheit 10 gelieferten Signale über elektrische Leitungen, die in die Leitung 11 integriert sein können, oder drahtlos an die Auswerte- und Steuereinheit 16 geliefert. Die Einheit 16, über die der Betrieb der Vorrichtung 1 gesteuert wird, weist einen Prozessor und einen mit dem Prozessor gekoppelten Speicher auf, mit deren Hilfe die empfangenen Signale zur Ermittlung eines Wertes für ein Qualitätsmaß des fertigen Pflastersteins, der sich nach Beendigung des Herstellungsprozesses aus dem Frischbeton-Pflasterstein 3 ergeben würde, unter Verwendung eines geeigneten Vorhersagemodells ausgewertet werden können. Die Einheit 16 weist dabei bevorzugt geeignete Eingabeeinrichtungen zur Eingabe von Parametern für die Auswertung auf.

Bevor die Vorrichtung 1 im Rahmen eines Herstellungsprozesses eines bestimmten Pflastersteinproduktes eingesetzt wird, muss die Einheit 16 mit den passenden Parametern für die Auswertung initialisiert werden. Zu diesem Zweck wird der Herstellungsprozess des Pflastersteinproduktes für eine Testcharge durchlaufen. In einem definierten Zwischenschritt in der Frischbetonphase des Herstellungsprozesses nach dem Formen und Verdichten in einer Steinformmaschine werden mit Hilfe der Transporteinrichtung 2 nacheinander mehrere Frischbeton-Pflastersteine 3 ergriffen und aus dem Herstellungsprozess entfernt. Die einzelnen Frischbeton-Pflastersteine 3 werden zu der Vorrichtung 1 transportiert und in der Messposition angeordnet, um die oben beschriebenen Messungen durchzuführen und für jeden Frischbeton-Pflasterstein 3 die entsprechenden Messwerte zu ermitteln. Die Messwerte werden in dem Speicher der Einheit 16 in der Weise gespeichert, dass eine spätere Zuordnung zu den einzelnen Frischbeton-Pflastersteinen 3 möglich ist. Jeder Frischbeton-Pflasterstein 3 wird nach seiner Messung und der Abschaltung des Unterdrucks in der Messeinheit 10 mit Hilfe der Transporteinrichtung 2 aus seiner Messposition wegtransportiert und wieder in den Herstellungsprozess eingeführt. Dabei wird der Frischbeton-Pflasterstein 3 durch Abschalten des Unterdrucks in der Basis 5 von der Transporteinrichtung 2 gelöst. Alternativ können die vermessenen Frischbeton-Pflastersteine 3 auch entsorgt werden, was geschehen muss, wenn sich die vermessenen Frischbeton-Pflastersteine 3 während der Messung Veränderungen erfahren. Im Falle der Entsorgung muss für das weitere Verfahren für jeden vermessenen Frischbeton-Pflasterstein 3 ein möglichst identischer Frischbeton-Pflasterstein vorhanden sein, der den Herstellungsprozess vollständig durchläuft. Solche Frischbeton-Pflastersteine mit annähernd identischen Eigenschaften lassen sich innerhalb derselben Charge mit identischer Mischung und an identischen Stellen in der Formmaschine herstellen.

Die vermessenen Frischbeton-Pflastersteine 3 bzw. die möglichst identischen Frischbeton-Pflastersteine 3 durchlaufen den Rest des Herstellungsprozesses vollständig. Anschließend, d.h. ca. 28 Tage nach der Vermessung, werden die aus den vermessenen Frischbeton-Pflastersteinen 3 bzw. den möglichst identischen Frischbeton-Pflastersteinen 3 entstandenen Festbeton-Pflastersteine auf ihre Qualität hin untersucht, indem Werte für ein oder mehrere Qualitätsmaße, wie zum Beispiel die Festigkeit oder die Rohdichte, bestimmt werden. Diese Messungen können mit Hilfe von separaten Messeinrichtungen durchgeführt werden. Es ist aber auch möglich, entsprechende Messeinrichtungen in die Vorrichtung 1 zu integrieren. In jedem Fall werden die gemessenen Qualitätswerte an die Einheit 16 geliefert, indem sie zum Beispiel manuell eingegeben werden, und in dem Speicher der Einheit 16 in der Weise gespeichert, dass sie den Messwerten des jeweils entsprechenden Frischbeton-Pflastersteins 3 zugeordnet sind. Der Prozessor der Einheit 16 kann dann mittels eines geeigneten Regressionsverfahrens ein Vorhersagemodell berechnen, das die Messwerte der Frischbeton-Pflastersteine 3 mit den Qualitätswerten der entsprechenden Festbeton-Pflastersteine in Beziehung setzt. Die vermessenen Frischbeton-Pflastersteine und ggf. die möglichst identischen Frischbeton-Pflastersteine stellen jeweils einen Satz von Kalibrierungs-Pflastersteinen dar.

Diese Initialisierung muss im allgemeinen für jedes einzelne Betonwarenprodukt, d.h. für eine bestimmte Rezeptur und bestimmte weitere Prozessparameter, wiederholt werden, da sich die Vorhersagemodelle im allgemeinen unterscheiden werden. Allerdings ist es in der Praxis möglich, die verschiedenen Betonwarenprodukte in Gruppen mit ausreichend ähnlichen Eigenschaften einzuteilen und für jede Gruppe ein einziges Vorhersagemodell zu verwenden, das für alle Gruppenmitglieder eine ausreichende Genauigkeit liefert. Im Extremfall ist es auch möglich ein einziges allgemeines Vorhersagemodell für alle Betonprodukte zu verwenden. Auch wenn in diesem Fall die Genauigkeit unter Umständen deutlich herabgesetzt ist, kann ein solches Vorhersagemodell für einfache und kostengünstige Abschätzungen ausreichen. Eine entsprechende Vorrichtung könnte in ihrem Aufbau vereinfacht werden.

Die Initialisierung kann von dem Benutzer selbst oder aber durch den Hersteller vorgenommen werden. So kann der Hersteller die Vorrichtung für eine Anzahl typischer Betonwarenprodukte vorinitialisieren.

Die verschiedenen Vorhersagemodelle werden in dem Speicher der Einheit 16 gespeichert. Sobald die Einheit 16 für ein bestimmtes Betonwarenprodukt initialisiert ist, ist es auf diese Weise möglich, an der Einheit 16 das passende Vorhersagemodell auszuwählen. Dann kann die Qualität des Herstellungsprozesses für zum Beispiel ein bestimmtes Pflastersteinprodukt kontrolliert werden, indem mit der Transporteinheit 3 dem Prozess vereinzelt Test-Frischbetonsteine in dem Zwischenschritt entnommen werden, der für die Erstellung des Vorhersagemodells verwendet wurde. Dazu muss die Bedienungsperson lediglich die Transporteinrichtung 2 auf den Test-Frischbetonstein 3 aufsetzen und die Vakuumpumpe zur Erzeugung von Unterdruck in der Basis 5 aktivieren. Nach Anordnung des Test-Frischbetonsteins 3 in der Messposition in der Vorrichtung 1 werden automatisch parallel die verschiedenen Messwerte ermittelt und an die Einheit 16 geliefert. Die Einheit 16 berechnet dann mit Hilfe des geeigneten Vorhersagemodells einen Wert für ein oder mehrere Qualitätsmaße des Endproduktes, das sich aus dem Test-Frischbetonstein 3 ergeben würde, wenn der Test-Frischbetonstein 3 wieder in den Herstellungsprozess eingeführt würde. Dieses Wiedereinführen ist tatsächlich ohne weiteres möglich, wenn die Messungen sehr schnell durchgeführt werden und der Test-Frischbetonstein 3 nicht verändert wird. Wird der Test-Frischbetonstein 3 wieder in den Herstellungsprozess eingeführt, so ist es von Vorteil, wenn an dem entsprechenden Endprodukt Werte für die Qualitätsmaße gemessen und an die Einheit 16 geliefert werden. In dieser Weise kann das Vorhersagemodell von der Einheit 16 während des normalen Betriebs weiter verbessert werden. Dasselbe kann auch erreicht werden, indem zu dem Test-Frischbetonstein 3 ein in der oben genannten Weise hergestellter möglichst identischer weiterer Test-Frischbetonstein 3 vorhanden ist, der den Herstellungsprozess vollständig durchläuft.

Die Vorrichtung kann auch noch kompakter ausgestaltet werden, indem anstatt dem Vorsehen der Waage 9 eine Wiegezelle in die Transporteinrichtung 2 eingebaut wird. Die Masse wird dann bestimmt, während das Test-Frischbetonprodukt an der Transporteinrichtung hängt. Wenn die Einheit 16 ebenfalls in die Transporteinrichtung 2 integriert wird oder die Messwerte drahtlos an eine separate Einheit 16 geliefert werden, kann die gesamte Vorrichtung, ggf. mit Ausnahme der Vakuumpumpen, als tragbare Vorrichtung ausgestaltet werden.

## Patentansprüche

1. Vorrichtung zur Qualitätskontrolle eines Prozesses zur Herstellung von Betonwarenprodukten, die angepasst ist, um Messungen an den Betonwarenprodukten in einem Zwischenschritt des Prozesses nach dem Formen und Verdichten in der Frischbetonphase durchzuführen, wobei die Vorrichtung (1) aufweist:
- eine Einrichtung (9, 14) zur Bestimmung eines Maßes für die Rohdichte eines Test-Frischbetonproduktes (3) aus dem Zwischenschritt des Prozesses und
- eine Einrichtung (6, 8, 10, 11, 13, 15) zur Bestimmung der Gaspermeabilität des Test-Frischbetonproduktes (3),
**dadurch gekennzeichnet, dass** die Einrichtung (6, 8, 10, 11, 13, 15) zur Bestimmung der Gaspermeabilität eine Kammer aufweist, die eine Öffnung (8, 13) hat, deren Ränder dichtend in Anlage an einen Bereich der Außenfläche des Test-Frischbetonproduktes (3) gebracht werden kann, um die Öffnung (8, 13) zu schließen, und dass die Einrichtung (6, 8, 10, 11, 13, 15) zur Bestimmung der Gaspermeabilität ferner eine Druckerzeugungseinrichtung, mit der in der Kammer ein Überdruck oder ein Unterdruck erzeugt werden kann, und eine Einrichtung (15) zur Bestimmung eines Maßes für den durch die Druckdifferenz bewirkten Gasdurchfluss durch das Test-Frischbetonprodukt (3) enthält,
wobei die Vorrichtung (1) so aufgebaut ist, dass das Test-Frischbetonprodukt (3) in einer einzigen Messposition angeordnet werden kann, in der gleichzeitig alle zur Bestimmung des Maßes für die Rohdichte und zur Bestimmung der Gaspermeabilität mit Hilfe der Einrichtung (9, 14) zur Bestimmung eines Maßes für die Rohdichte und der Einrichtung (6, 8, 10, 11, 13, 15) zur Bestimmung der Gaspermeabilität erforderlichen Messwerte ermittelt werden können, und dass das Test-Frischbetonprodukt (3) durch Unterdruck in der Messposition fixiert werden kann.

2. Vorrichtung nach Anspruch 1, bei der die Druckerzeugungseinrichtung angepasst ist, um in der durch das Test-Frischbetonprodukt (3) abgeschlossenen Kammer einen Unterdruck zu erzeugen.

3. Vorrichtung nach Anspruch 2, bei der die Druckerzeugungseinrichtung eine Vakuumpumpe umfasst und bei der die Einrichtung (6, 8, 10, 11, 13, 15) zur Bestimmung der Gaspermeabilität eine Druckmeßeinrichtung (15) zur Bestimmung des Druckes in der Kammer aufweist.

4. Vorrichtung nach Anspruch 3, die ferner eine Zeitmesseinrichtung enthält, mit der die Geschwindigkeit des Druckanstiegs nach Deaktivierung der Vakuumpumpe gemessen werden kann.

5. Vorrichtung nach einem der Ansprüche 2 bis 4, die ferner eine Transporteinrichtung (2) zum Anheben und Transportieren des Test-Frischbetonproduktes (3) aufweist, wobei die Kammer einen Teil der Transporteinrichtung (2) bildet und das Test-Frischbetonprodukt (3) angehoben und transportiert werden kann, wenn in der Kammer ein Unterdruck erzeugt wird.

6. Vorrichtung nach einem der Ansprüche 1 bis 5, bei der die Einrichtung (6, 8, 10, 11, 13, 15) zur Bestimmung der Gaspermeabilität angepasst ist, um die Permeabilität gegenüber Luft zu bestimmen.

7. Vorrichtung nach einem der Ansprüche 1 bis 6, bei der die Einrichtung (9, 14) zur Bestimmung eines Maßes für die Rohdichte des Test-Frischbetonproduktes (3) eine Einrichtung (14) zur Bestimmung einer geometrischen Abmessung des Test-Frischbetonproduktes (3) und eine Einrichtung (9) zur Bestimmung der Masse des Test-Frischbetonproduktes (3) aufweist.

8. Vorrichtung nach Anspruch 7, bei der die Einrichtung (14) zur Bestimmung einer geometrischen Abmessung eine optische Messeinrichtung ist, mit der die geometrische Abmessung mit einem geeigneten optischen Messverfahren gemessen werden kann.

9. Vorrichtung nach Anspruch 8, bei der die Einrichtung (14) zur Bestimmung einer geometrischen Abmessung einen Laser aufweist, mit dessen Hilfe die geometrische Abmessung bestimmt werden kann.

10. Vorrichtung nach einem der Ansprüche 7 bis 9, bei der die Einrichtung (14) zur Bestimmung einer geometrischen Abmessung angepasst ist, um die Höhe eines quaderförmigen Betonwarenproduktes zu bestimmen.

11. Vorrichtung nach einem der Ansprüche 1 bis 10, die ferner eine Auswerteeinrichtung (16) aufweist, die mit der Einrichtung (9, 14) zur Bestimmung eines Maßes für die Rohdichte und der Einrichtung (6, 8, 10, 11, 13, 15) zur Bestimmung der Gaspermeabilität gekoppelt und angepasst ist, um die von diesen gelieferten Messwerte für ein Test-Frischbetonprodukt (3) aus dem Zwischenschritt des Prozesses zu empfangen und anhand eines vorbestimmten Vorhersagemodells, das die Messwerte von Frischbetonprodukten aus dem Zwischenschritt des Prozesses mit einem Qualitätsmaß der entsprechenden Festbetonprodukte in Beziehung setzt, einen geschätzten Wert für das Qualitätsmaß des Festbetonproduktes zu ermitteln, das sich im weiteren Prozessverlauf aus dem vermessenen Test-Frischbetonprodukt (3) ergibt.

12. Vorrichtung nach einem der Anspruch 11, bei der die Auswerteeinrichtung (16) einen Speicher aufweist, in dem die Messwerte für einen Satz von Kalibrierungs-Frischbetonprodukten aus dem Zwischenschritt des Prozesses gespeichert werden können, und die ferner eine Eingabeeinrichtung zur Eingabe von Werten für das Qualitätsmaß für jedes der Festbetonprodukte, die sich aus den Kalibrierungs-Frischbetonprodukten ergeben, oder für Festbetonprodukte, die sich aus einem zweiten Satz von Kalibrierungs-Frischbetonprodukten ergeben, der in derselben Weise hergestellt wurde wie der erste Satz, wobei die Auswerteeinrichtung angepasst ist, um die eingegebenen Werte für das Qualitätsmaß in dem Speicher zu speichern und einen Zusammenhang zwischen den Werten für das Qualitätsmaß und den Messwerten zu ermitteln.

## Claims

1. An apparatus for quality control of a process for producing concrete goods products, which apparatus is adapted to carry out measurements on the concrete goods products in an intermediate step of the process after shaping and compacting in the fresh concrete phase, wherein the apparatus (1) comprises:
- a means (9, 14) for determining a measure of the bulk density of a test fresh concrete product (3) from the intermediate step of the process, and
- a means (6, 8, 10, 11, 13, 15) for determining the gas permeability of the test fresh concrete product (3)
**characterized in that** the means (6, 8, 10, 11, 13, 15) for determining the gas permeability comprises a chamber, that has an opening (8, 13) the edges of which can be sealingly placed against an area of the outer surface of the test fresh concrete product (3) in order to close the opening (8, 13), and **in that** the means (6, 8, 10, 11, 13, 15) for determining the gas permeability further comprises a pressure generation means, with which a positive pressure or a negative pressure can be generated within the chamber, and a means (15) for determining a measure of the gas flow through the test fresh concrete product (3) caused by the pressure difference,
wherein the apparatus (1) is constructed such that the test fresh concrete product (3) can be disposed in a single measurement position in which all measurement values required for determining the measure of the bulk density and for determining the gas permeability by means of the means (9, 14) for determining a measure of the bulk density and the means (6, 8, 10, 11, 13, 15) for determining the gas permeability can be obtained simultaneously, and that the test fresh concrete product (3) can be fixed in the measurement position by negative pressure.

2. The apparatus according to claim 1, wherein the pressure generation means is adapted to generate a negative pressure within the chamber closed by the test fresh concrete product (3).

3. The apparatus according to claim 2, wherein the pressure generation means comprises a vacuum pump and wherein the means (6, 8, 10, 11, 13, 15) for determining the gas permeability comprises a pressure measurement means (15) for determining the pressure within the chamber.

4. The apparatus according to claim 3, further comprising a time measurement means with which the rate of the pressure increase following deactivation of the vacuum pump can be measured.

5. The apparatus according to any of claims 2 to 4, further comprising a transport means (2) for lifting and transporting the test fresh concrete product (3), wherein the chamber constitutes a part of the transport means (2) and the test fresh concrete product (3) can be lifted and transported when a negative pressure is generated within the chamber.

6. The apparatus according to any of claims 1 to 5, wherein the means (6, 8, 10, 11, 13, 15) for determining the gas permeability is adapted to determine the permeability with respect to air.

7. The apparatus according to any of claims 1 to 6, wherein the means (9, 14) for determining a measure of the bulk density of the test fresh concrete product (3) comprises a means (14) for determining a geometric dimension of the test fresh concrete product (3) and a means (9) for determining the mass of the test fresh concrete product (3).

8. The apparatus according to claim 7, wherein the means (14) for determining a geometric dimension is an optical measurement means by means of which the geometric dimension can be measured with a suitable optical measurement method.

9. The apparatus according to claim 8, wherein the means (14) for determining a geometric dimension comprises a laser by means of which the geometric dimension can be determined.

10. The apparatus according to any of claims 7 to 9, wherein the means (14) for determining a geometric dimension is adapted to determine the height of a rectangular concrete goods product.

11. The apparatus according to any of claims 1 to 10, further comprising an evaluation means (16) that is coupled to the means (9, 14) for determining a measure of the bulk density and the means (6, 8, 10, 11, 13, 15) for determining the gas permeability, and that is adapted to receive the measurement values for a test fresh concrete product (3) from the intermediate step of the process provided by these means and to determine on the basis of a predetermined predictive model, that relates the measurement values of fresh concrete products from the intermediate step of the process to a quality measure of the corresponding hardened concrete products, an estimated value for the quality measure of the hardened concrete product that results in the further course of the process from the measured test fresh concrete product (3).

12. The apparatus according to claim 11, wherein the evaluation means (16) comprises a memory in which the measurement values for a set of calibration fresh concrete products from the intermediate step of the process can be stored, and that further comprises an input means for inputting values for the quality measure for each of the hardened concrete products, that result from the calibration fresh concrete products, or for hardened concrete products, that result from a second set of calibration fresh concrete products that has been produced in the same manner as the first set, wherein the evaluation means is adapted to store the inputted values for the quality measure in the memory and to determine a relationship between the values for the quality measure and the measurement values.

## Revendications

1. Dispositif pour le contrôle qualitatif d'un procédé de fabrication de produits en béton, lequel est adapté pour effectuer des mesures sur les produits en béton dans une étape intermédiaire de procédé postérieure au moulage et au compactage dans la phase de béton frais, lequel dispositif (1) présente :
- un dispositif (9, 14) pour déterminer une mesure de la densité d'un produit en béton frais test (3) issu de l'étape intermédiaire du procédé et
- un dispositif (6, 8, 10, 11, 13, 15) pour déterminer la perméabilité aux gaz du produit en béton frais test (3),
**caractérisé en ce que** le dispositif (6, 8, 10, 11, 13, 15) pour déterminer la perméabilité aux gaz présente une chambre pourvue d'une ouverture (8, 13) dont les bords peuvent être amenés en contact étanche avec une zone de la surface extérieure du produit en béton frais test (3) pour fermer l'ouverture (8, 13), et **en ce que** le dispositif (6, 8, 10, 11, 13, 15) pour déterminer la perméabilité aux gaz comprend également un dispositif de production de pression, avec lequel une surpression ou une dépression peut être produite dans la chambre, et un dispositif (15) pour déterminer une mesure du débit de gaz provoqué par la différence de pression à travers le produit en béton frais test (3), le dispositif (1) étant conçu de façon que le produit en béton frais test (3) puisse être disposé dans une seule position de mesure permettant de connaître en même temps toutes les valeurs de mesure nécessaires pour déterminer la mesure de la densité et pour déterminer la perméabilité aux gaz à l'aide du dispositif (9, 14) pour déterminer une mesure de la densité et à l'aide du dispositif (6, 8, 10, 11, 13, 15) pour déterminer la perméabilité aux gaz, et **en ce que** le produit en béton frais test (3) peut être immobilisé dans la position de mesure par dépression.

2. Dispositif selon la revendication 1, dans lequel le dispositif de production de pression est apte à produire une dépression dans la chambre fermée par le produit en béton frais test (3).

3. Dispositif selon la revendication 2, dans lequel le dispositif de production de pression comprend une pompe à vide et dans lequel le dispositif (6, 8, 10, 11, 13, 15) pour déterminer la perméabilité aux gaz comporte un dispositif de mesure de pression (15) pour déterminer la pression dans la chambre.

4. Dispositif selon la revendication 3, qui contient également un dispositif de mesure du temps permettant de mesurer la vitesse de la montée en pression après désactivation de la pompe à vide.

5. Dispositif selon une des revendications 2 à 4, qui comporte également un dispositif de transport (2) pour soulever et transporter le produit en béton frais test (3), la chambre formant une partie du dispositif de transport (2), et le produit en béton frais test (3) pouvant être soulevé et transporté lorsqu'une dépression est produite dans la chambre.

6. Dispositif selon une des revendications 1 à 5, dans lequel le dispositif (6, 8, 10, 11, 13, 15) pour déterminer la perméabilité aux gaz est apte à déterminer la perméabilité par rapport à l'air.

7. Dispositif selon une des revendications 1 à 6, dans lequel le dispositif (9, 14) pour déterminer une mesure de la densité du produit en béton frais test (3) comporte un dispositif (14) pour déterminer une dimension géométrique du produit en béton frais test (3) et un dispositif (9) pour déterminer la masse du produit en béton frais test (3).

8. Dispositif selon la revendication 7, dans lequel le dispositif (14) pour déterminer une dimension géométrique est un dispositif de mesure optique permettant de mesurer la dimension géométrique par un procédé de mesure optique approprié.

9. Dispositif selon la revendication 8, dans lequel le dispositif (14) pour déterminer une dimension géométrique présente un laser permettant de déterminer la dimension géométrique.

10. Dispositif selon une des revendications 7 à 9, dans lequel le dispositif (14) pour déterminer une dimension géométrique est apte à déterminer la hauteur d'un produit en béton de forme parallélépipédique.

11. Dispositif selon une des revendications 1 à 10, qui comporte également un dispositif d'analyse (16) couplé et adapté au dispositif (9, 14) pour déterminer une mesure de la densité et au dispositif (6, 8, 10, 11, 13, 15) pour déterminer la perméabilité aux gaz, afin de recevoir les valeurs de mesure fournies par ceux-ci pour un produit en béton frais test (3) issu de l'étape intermédiaire du procédé et de déterminer, à l'aide d'un modèle prévisionnel prédéterminé mettant en relation les valeurs de mesure de produits en béton frais issus de l'étape intermédiaire du procédé avec une mesure qualitative des produits en béton durci correspondants, une valeur estimée de la mesure qualitative du produit en béton durci qui résulte du produit en béton frais test (3) mesuré dans la suite du procédé.

12. Dispositif selon la revendication 11, dans lequel le dispositif d'analyse (16) comporte une mémoire où peuvent être enregistrées les valeurs de mesure d'un jeu étalon de produits en béton frais issu de l'étape intermédiaire du procédé, et qui comporte également un dispositif de saisie pour saisir des valeurs de mesure qualitative pour chacun des produits en béton durci qui résultent des produits étalons en béton frais ou pour des produits en béton durci qui résultent d'un second jeu étalon de produits en béton frais fabriqué de la même manière que le premier jeu, le dispositif d'analyse étant apte à enregistrer dans la mémoire les valeurs saisies pour la mesure qualitative et à déterminer une relation entre les valeurs de mesure qualitative et les valeurs mesurées.
